# EUROPEAN PATENT APPLICATION

(11) **EP 1 985 704 A1**
(43) Date of publication of application: **29.10.2008**
(21) Application number: 07714819.5
(22) Date of filing: 16.02.2007
(51) Int. Cl.: C12N 15/29, A01H 5/00, C12N 1/15, C12N 1/19, C12N 1/21, C12N 5/10, C12N 9/10

(54) **FLAVONOID GLYCOSYLTRANSFERASE AND UTILIZATION THEREOF**

(30) Priority: 17.02.2006 JP 2006041280
(71) Applicant: International Flower Developments Proprietary Limited, Bundoora Victoria 3083 (AU)
(72) Inventor: TANAKA, Yoshikazu, Mishima-gun, Osaka 618-8503 (JP); KATSUMOTO, Yukihisa, Mishima-gun, Osaka 618-8503 (JP); FUKUI, Yuko, Mishima-gun, Osaka 618-8503 (JP); MIZUTANI, Masako, Mishima-gun, Osaka 618-8503 (JP); TOGAMI, Junichi, Mishima-gun, Osaka 618-8503 (JP)
(74) Representative: Vossius & Partner
(86) International application number: PCT/JP2007/053342
(87) International publication number: WO 2007/094521

(57) **Abstract**

A novel flavonoid glucosyltransferase originating from rose, a nucleic acid encoding that enzyme, for example, a flavonoid glucosyltransferase having an amino acid sequence indicated in SEQ ID NO. 2 or SEQ ID NO. 4, a nucleic acid encoding that enzyme, a method for changing the color of a rose flower using that nucleic acid, and a rose plant capable of changing color, are provided.

## Description

### TECHNICAL FIELD

The present invention relates to the gene of an enzyme that glycosylates flavonoids and a method for using the same.

### BACKGROUND ART

Since flowers exhibiting new traits are always considered to have significant value in the ornamental plant industry, the development of such ornamental plants is industrially important. Flower color is the most important trait of ornamental plants. Cultivars with various colors have been bred by breeding mainly with using of hybridization breeding. However, in the case of breeding by hybridization breeding, since available genetic resources are limited to closely related plants that can be hybridized, there are many cases in which plant color is limited, and it is rare that a single plant species has cultivars exhibiting all colors. For example, blue roses and carnations, yellow morning glories and geraniums etc. were unable to be developed by breeding with using hybridization breeding.

The main components of flower color are a group of flavonoid compounds generically referred to as anthocyanins. A wide range of anthocyanins are known to exist in plants, and many of their structures have already been determined. The color of anthocyanins is primarily dependent upon their structures. Anthocyanins known to be contained in roses are cyanidin 3,5-diglucoside, pelargonidin 3,5-diglucoside, cyanidin 3-glucoside, pelargonidin 3-glucoside, peonidin 3,5-diglucoside and peonidin 3-glucoside. These determine the colors of orange to red in rose flowers. A blue compound generically referred to as rosacyanin is contained in some roses, and rosacyanin is a derivative of cyanidin, which is a type of anthocyanidin (Tetrahedron Letters (2002) 43, 2637-2639, Japanese Unexamined Patent Publication No. 2002-201372, Tetrahedron (2006) 62, 9661-9670).

Considerable research has been conducted on enzymes and genes involved in the biosynthesis of anthocyanins (Plant Cell, Tissue and Organ Culture (2005) 80, 1-24). There are some examples that the structure of anthocyanins are modified to change flower color by introducing and expressing these genes in plants (Plant Cell, Tissue and Organ Culture (2005) 80, 1-24). The biosynthesis of anthocyanins leading to anthocyanidin 3-glucosides is conserved among nearly all flowering plants, and anthocyanidins are typically first modified with a sugar such as glucose at the 3 position (Plant Cell, Tissue and Organ Culture (2005) 80, 1-24). Alternatively, galactose is added instead of glucose depending on the plant species, resulting in the accumulation of anthocyanidin 3-galactosides.

However, in roses, which are industrially important horticultural plants, anthocyanidin 3-glucosyltransferase activity has not been detected, while, differing from other flowering plants, anthocyanidin 5,3-glucosyltransferase (A5,3GT) is present. Namely, a glucose is reported to transfer to the 3 position of an anthocyanidin after having transferred to the 5 position (Nature (2005) 435, 757-758). On the other hand, the blue rosacyanin present in some roses shows structure containing cyanidin. Although it is not known how this is biosynthesized from cyanidin, based on the structure of rosacyanin, it is clear that the reaction in which a sugar is transferred to cyanidin is unnecessary for the biosynthesis of rosacyanin.

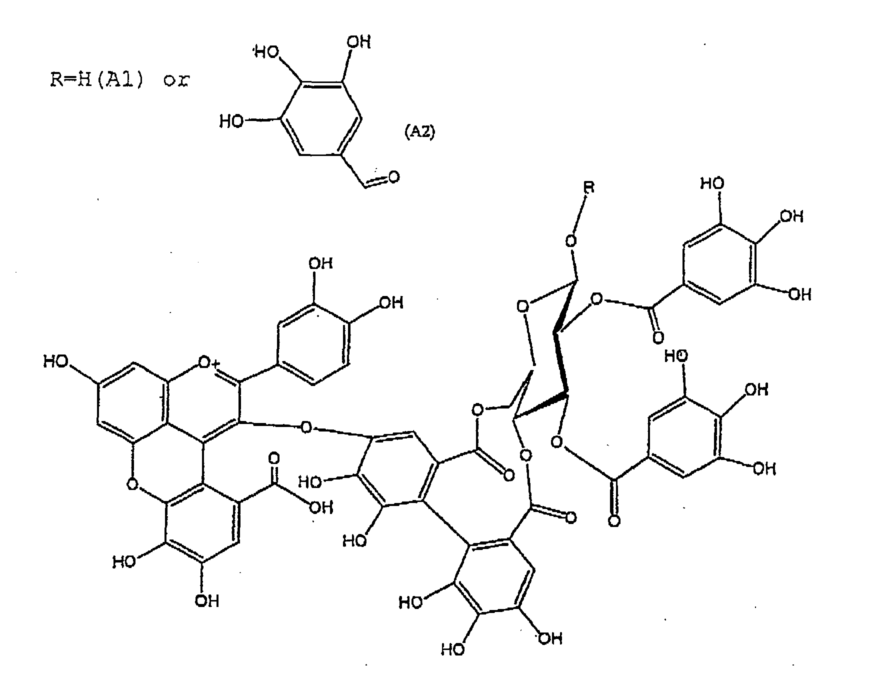

On the basis of the above, it is suggested that the presence of glucosyltransferase activity toward anthocyanidins is important for determining whether anthocyanin or rosacyanin is biosynthesized in rose petals, namely if anthocyanidin glucosyltransferase activity is present, anthocyanin is synthesized, while if anthocyanidin glucosyltransferase activity is not present, rosacyanin is synthesized. On the basis of previous findings (Nature (2005) 435, 757-758), in the case of roses, it has been suggested that the anthocyanidin glucosyltransferase thereof is not anthocyanidin 3-glucosyltransferase, but rather anthocyanidin 5,3-glucosyltransferase (A5,3GT).

Enzymes that transfer sugars to various compounds including flavonoids use sugar donors such as UDP-glucose or UDP-rhamnose and catalyze reactions that transfer a sugar to a hydroxyl group to a specific or non-specific location of a compound. Such glucosyltransferases form a glucosyltransferase family in the plant genome, and more than 100 kinds of molecular species are known to exist for a single species (Glycobiology (2003) 13, 139-145).

Glucosyltransferases, which transfer sugars to flavonoids, including anthocyanidins, also belong to this glucosyltransferase family. There are a comparatively large number of findings describing enzymes that transfer sugars to flavonoids within this family. In many cases, enzymes that transfer sugars to flavonoids catalyze a reaction that transfers a sugar to a hydroxyl group at a specific position on the flavonoid, and the amino acid sequences of enzymes that transfer sugars to the same position are similar irrespective of plant species.

Reactions that transfer a sugar to the 3 position or the 5 position of flavonoids, including anthocyanins, have thus far been known to be catalyzed by different enzymes such as flavonoid (or anthocyanidin) 3-glucosyltransferase (A3GT) and anthocyanin 5-glucosyltransferase (A5GT), respectively, and genes that encode these enzymes have been obtained from several plants. In addition, each of these enzymes respectively form sub-families (Plant Mol. Biol. (2002) 48, 401-11, Current Opinions in Plant Biology (2005) 8, 1-10). In addition, the amino acid sequences of enzymes transferring sugars to the 3' position and the 7 position of flavonoids exhibit a comparatively high degree of homology, and flavonoid 7-glucosyltransferase, which transfers sugar to the 7 position of flavonoids (Planta. (2000) 210, 1006-1013) and gentian anthocyanin 3'-glucosyltransferase, which transfers sugar to the 3' position of anthocyanins (Plant Physiol. (2003) 132, 1652-1663), belong to the same subfamily. On the other hand, rose A5,3GT belongs to a different sub-family.

By the way, some cultivars of roses are known to change color after they have bloomed, examples of which include cultivars such as Masquerade and EHIGASA. Although these cultivars have a yellow to cream color from bud to the early stages of blooming, during blooming progresses, flower color changes to red or pink. In these color-changing roses, anthocyanins are synthesized in the bud stage similar to other rose cultivars. However, since their flower colors change to a red or pink color, there is believed to probably be a stage during which anthocyanins are again actively synthesized even after blooming. The Masquerade cultivar produced in 1949 is said to be the first cultivar to change color after blooming (Encyclopedia of Roses, Japan Broadcast Publishing Co., Ltd., ISBN 4-14-645776-9). Such traits are inherited in subsequent generations by hybridization breeding and although new cultivars have been developed, coloring following blooming is limited to red or pink.

In addition, A3GT gene has recently been reported to be expressed accompanying anthocyanin synthesis in cultured cells of a similarly color-changing rose cultivar known as Charleston (Plant Biotechnology (2006) 23, 379-385).

On the basis of the current level of technology, it is possible to produce a genetically recombinant rose where an exogenous gene is overexpressed or an endogenous gene is inhibited therein.
Patent Document 1: Japanese Unexamined Patent Publication No. 2002-201372
Non-Patent Document 1: Tetrahedron Letters (2002) 43, 2637-2639
Non-Patent Document 2: Plant Cell, Tissue and Organ Culture (2005) 80, 1-24
Non-Patent Document 3: Nature (2005) 435, 757-758
Non-Patent Document 4: Glycobiology (2003) 13, 139-145
Non-Patent Document 5: Plant Mol. Biol. (2002) 48, 401-11
Non-Patent Document 6: Planta. (2000) 210, 1006-1013
Non-Patent Document 7: Plant J. (2003) 132, 1652-1663
Non-Patent Document 8: Tetrahedron (2006) 62, 9661-9670
Non-Patent Document 9: Plant Biotechnology (2006) 23, 379-385

### DISCLOSURE OF THE INVENTION

An enzyme, and gene thereof, which transfers a sugar to the 3 position (before transferring to the 5 position) of anthocyanins in roses, or an enzyme, and gene thereof, which transfers a sugar only or preferentially to the 3 position, are not known, and such enzymes are not considered to exist (Nature (2005) 435, 757-758). If such enzymes or genes were able to be obtained, it would be possible to alter the biosynthesis pathway of anthocyanin and rosacyanin in roses, thereby clearly making it possible to alter the flower color of roses. In addition, as is observed in roses that change color accompanying blooming, if it were possible to acquire a gene involved in anthocyanin synthesis involved in color change after blooming (including expression regulating moieties such as the promoter of that gene), it would be possible to develop a rose that changes color after blooming by molecular breeding using that gene.

Thus, the present invention provides a nucleic acid encoding a protein originating in family *Rosa* ceae genus rosa having activity that transfers a sugar to a 3-position hydroxyl group of a flavonoid. In particular, the flavonoid is anthocyanidin or flavonol.

In addition, the present invention provides a nucleic acid encoding a protein having an amino sequence indicated in SEQ ID NO. 2 or SEQ ID NO. 4, which has activity that transfers a sugar to a 3-position hydroxyl group of a flavonoid.

Moreover, the present invention provides a nucleic acid encoding a protein having an amino acid sequence that has been modified by addition, deletion and/or substitution with other amino acids to one or a plurality of amino acids in an amino acid sequence indicated in SEQ ID NO. 2 or SEQ ID NO. 4, and having activity that transfers a sugar to a 3-position hydroxyl group of a flavonoid, and more specifically, provides a nucleic acid encoding a protein having an amino acid sequence that has been modified by addition, deletion and/or substitution with other amino acids to one or several amino acids in an amino acid sequence indicated in SEQ ID NO. 2 or SEQ ID NO. 4, and having activity that transfers a sugar to a 3-position hydroxyl group of a flavonoid.

In addition, the present invention provides a nucleic acid encoding a protein having sequence identity of at least 60% with respect to an amino acid sequence indicated in SEQ ID NO. 2 or SEQ ID NO. 4 and having activity that transfers a sugar to a 3-position hydroxyl group of a flavonoid; for example, a nucleic acid encoding a protein having sequence identity of at least 80% with respect to an amino acid sequence indicated in SEQ ID NO. 2 or SEQ ID NO. 4 and having activity that transfers a sugar to a 3-position hydroxyl group of a flavonoid; and more specifically, a nucleic acid encoding a protein having sequence identity of at least 90% with respect to an amino acid sequence indicated in SEQ ID NO. 2 or SEQ ID NO. 4 and having activity that transfers a sugar to a 3-position hydroxyl group of a flavonoid.

Moreover, the present invention provides a nucleic acid encoding a protein having an amino acid sequence encoded by a nucleic acid that hybridizes under moderate stringent conditions with a nucleic acid having a nucleotide sequence of a coding region indicated in SEQ ID NO. 1 or SEQ ID NO. 3, and having activity that transfers a sugar to a 3-position hydroxyl group of a flavonoid; and more specifically, provides a nucleic acid encoding a protein having an amino acid sequence encoded by a nucleic acid that hybridizes under high stringent conditions with a nucleic acid having a nucleotide sequence of a coding region indicated in SEQ ID No. 1 or SEQ ID NO. 3, and having activity that transfers a sugar to a 3-position hydroxyl group of a flavonoid.

A typical gene of the present invention is a nucleic acid containing a nucleotide sequence of a coding region indicated in SEQ ID NO. 1 or SEQ ID NO. 3.

A nucleic acid of the present invention is DNA or RNA, and particularly DNA. Moreover, the present invention provides a vector containing this DNA and a host that has been transformed by this vector.

Moreover, the present invention provides a plant into which any of the nucleic acids described above has been inserted, a progeny thereof having identical properties thereto, or tissues or vegetatively propagated plants thereof; a plant in which any of the nucleic acids described above has been inserted and for which flower color has been altered, progeny thereof having identical properties thereto, or tissue or vegetative growth body thereof; and, a cut flower of the above plants or progeny thereof having identical properties thereto.

Moreover, the present invention provides a protein encoded by any of the various nucleic acids described above. In addition, the present invention provides a method of producing a protein having activity that transfers a sugar to a 3-position hydroxyl group of a flavonoid, by culturing or growing the host, and then harvesting the protein from the host.

### BRIEF DESCRIPTION OF THE DRAWINGS

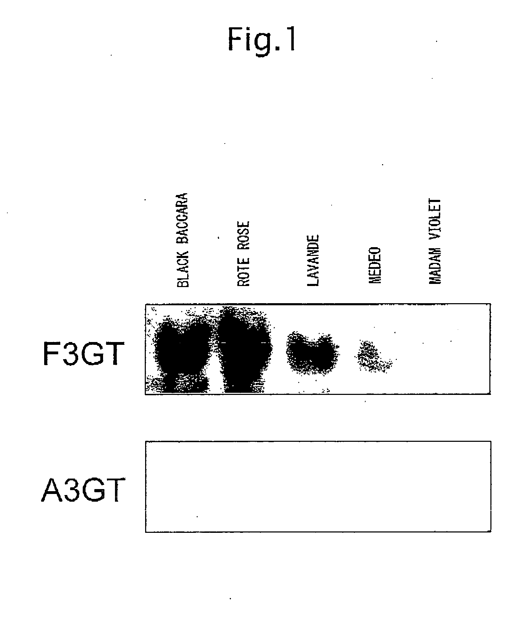

Fig. 1 shows the expression of F3GT gene and A3GT gene in the petals of buds of various cultivars of roses. The figure is resulted from Northern hybridization of RNA obtained from the petals of buds in which anthocyanin synthesis is active using a DIG RNA probe originating in the F3GT sequence or A3GT sequence.

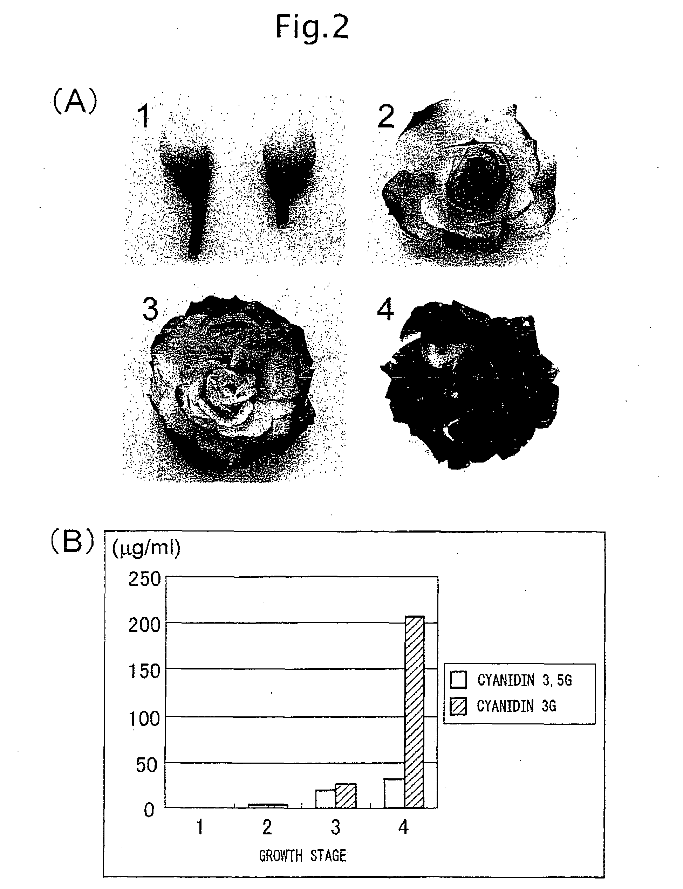

Fig. 2 shows the state of petals at each develpomental stage of a rose (cultivar: Ehigasa) (when flower color is changing from orange-yellow to red) (A), and the amount of anthocyanin present in the petals at each growth stage (B).

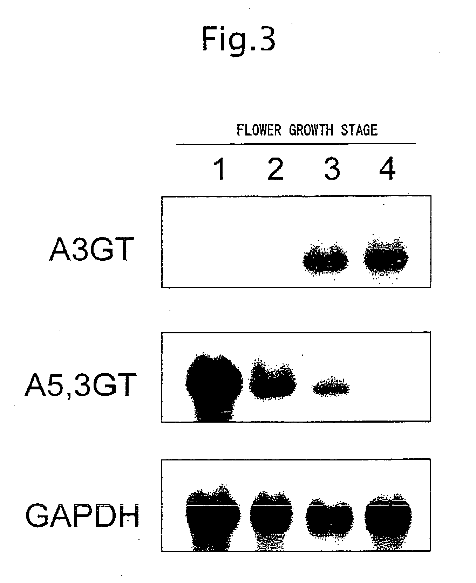

Fig. 3 shows the expression of A3GT gene and A5,3GT gene in petals at each developmental stage of a rose (cultivar: Ehigasa). Expression of A3GT gene, A5,3GT gene and GAPDH gene at each growth stage were investigated by Northern hybridization. DIG-labeled RNA probes originating in each gene sequence were used. The flower growth stages of 1 to 4 correspond to growth stages 1 to 4 in Fig. 2.

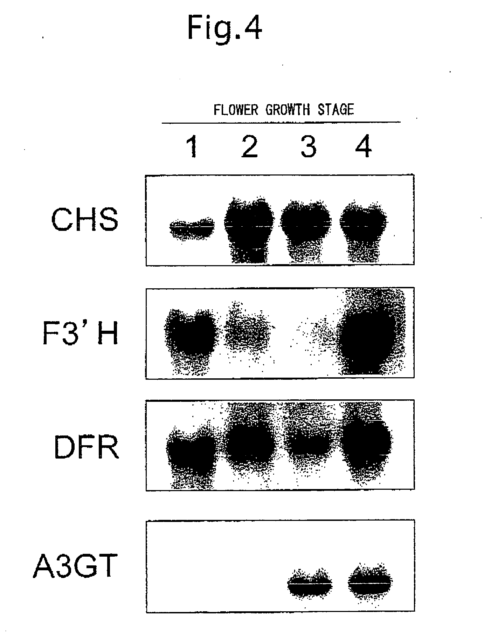

Fig. 4 shows the expression of CHS gene, F3'H gene, DFR gene and A3GT gene in petals at each developmental stage of a rose (cultivaer: Ehigasa). Expression of CHS gene, F3'H gene, DFR gene and A3GT gene at each develpomental stage was investigated by Northern hybridization. DIG-labeled RNA probes originating in each gene sequence were used. The flower growth stages of 1 to 4 correspond to growth stages 1 to 4 in Fig. 2.

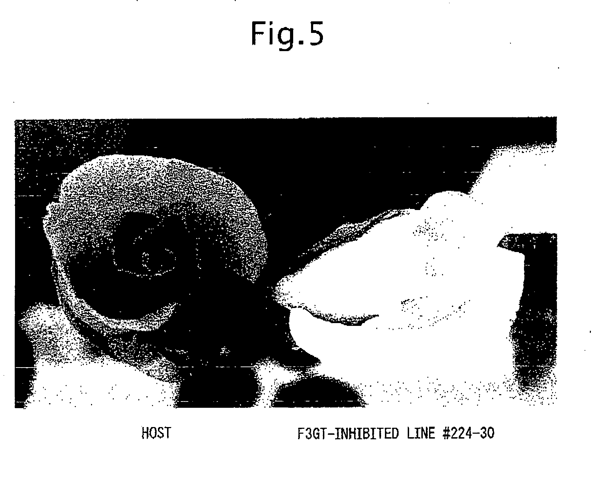

Fig. 5 shows changes in flower color in the case of introduction of pSFL224, which controls expression of F3GT, into a rose host (cultivar: Lavande). The reddish-violet color of the host became considerably lighter in the transformants.

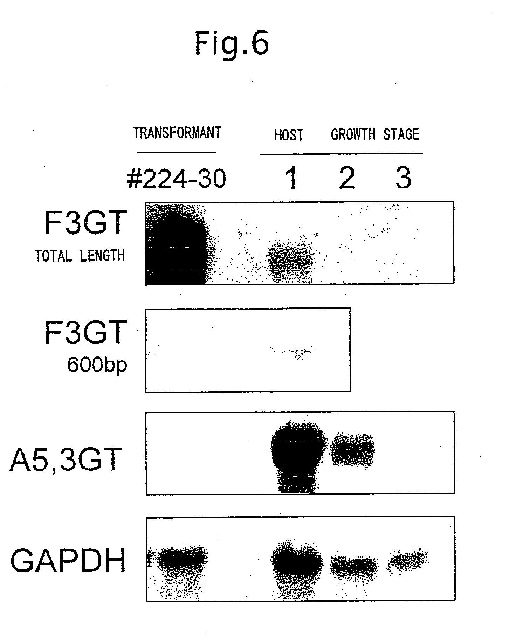

Fig. 6 shows the expression of F3GT gene and A5,3GT gene in a rose host (cultivar: Lavande) and an F3GT-inhibited transformant (same as Fig. 5). Expression of F3GT gene, A5,3GT gene and GAPDH gene in the Lavande host and F3GT-inhibited transformant was investigated by Northern hybridization. DIG-labeled RNA probes originating in each gene sequence were used. A portion present only in endogenous F3GT gene was used for the F3GT 600 bp probe. The flower developmental stages of 1 to 4 correspond to developmental stages 1 to 4 in Fig. 2.

### BEST MODE FOR CARRYING OUT THE INVENTION

A protein encoded by the nucleic acid of the present invention having activity that transfers a sugar to a 3-position hydroxyl group of a flavonoid most typically has an amino acid sequence indicated in SEQ ID NO. 2 or SEQ ID NO. 4. However, it is known in the technical field of enzymes that an essential region involved in enzyme activity or substrate specificity and the like and a non-essential region for enzyme activity and other properties are present within the enzyme protein, and the enzyme activity and other important properties are maintained even if the amino acid sequence in the non-essential region is modified by addition or deletion of amino acids or substitution of amino acids with other amino acids.

Thus, the present invention provides not only a nucleic acid encoding an enzyme having an amino acid sequence indicated in SEQ ID NO. 2 or SEQ ID NO. 4, but also provides a protein having an amino acid sequence that has been modified by addition, deletion and/or substitution with other amino acids to one or a plurality of amino acids in an amino acid sequence indicated in SEQ ID NO. 2 or SEQ ID NO. 4, and having activity that transfers a sugar to a 3-position hydroxyl group of a flavonoid, as well as a nucleic acid encoding that protein. For example, the present invention provides a protein having an amino acid sequence that has been modified by addition, deletion and/or substituted with other amino acids to one or several amino acids in an amino acid sequence indicated in SEQ ID NO. 2 or SEQ ID NO. 4, and having activity that transfers a sugar to a 3-position hydroxyl group of a flavonoid, and a nucleic acid encoding that protein. The number of amino acids subjected to modification is 50 or less, for example 40 or less, 30 or less or 20 or less, and typically 10 or less.

Enzyme activity is maintained even if modifications are added to a naturally-occurring amino acid sequence as was previously described. As will be described later, the amino acid sequence of the enzyme of the present invention only has amino acid sequence identity of less than 60% with an amino acid sequence having the highest sequence identity of a known enzyme having similar activity. Thus, the present invention provides a protein having sequence identity of at least 60% with respect to an amino acid sequence indicated in SEQ ID NO. 2 or SEQ ID NO. 4 and having activity that transfers a sugar to a 3-position hydroxyl group of a flavonoid, and a nucleic acid encoding that protein; a protein having sequence identity of at least 80% with respect to an amino acid sequence indicated in SEQ ID NO. 2 or SEQ ID NO. 4 and having activity that transfers a sugar to a 3-position hydroxyl group of a flavonoid, and a nucleic acid encoding that protein; and more limitedly, a protein having sequence identity of at least 90% with respect to an amino acid sequence indicated in SEQ ID NO. 2 or SEQ ID NO. 4 and having activity that transfers a sugar to a 3-position hydroxyl group of a flavonoid, and a nucleic acid encoding that protein.

It is known that if a gene encoding a certain enzyme is cloned, a gene encoding a different enzyme having similar activity can be cloned by using a probe and primers designed on the basis of the sequence data of that gene. Thus, the present invention also provides a protein having an amino acid sequence encoded by a nucleic acid that hybridizes under moderate stringent conditions with respect to a nucleic acid having a base sequence of a coding region indicated in SEQ ID NO. 1 or SEQ ID NO. 3 and having activity that transfers a sugar to a 3-position hydroxy group of a flavonoid, and a nucleic acid encoding that protein; and more specifically, a protein having an amino acid sequence encoded by a nucleic acid that hybridizes under high stringent conditions with respect to a nucleic acid having a base sequence of a coding region indicated in SEQ ID NO. 1 or SEQ ID NO. 3 and having activity that transfers a sugar to a 3-position hydroxy group of a flavonoid, and a nucleic acid encoding that protein.

The aforementioned moderate stringent conditions refer to hybridizing overnight at 37°C in 5xSSC containing 30% formaldehyde followed by washing at 55°C in 5xSSC containing 1% SDS, while the high stringent conditions refer to hybridizing overnight at 42°C in 5xSSC containing 50% formaldehyde followed by washing at 65°C in 2xSSC containing 1% SDS.

In addition, the present invention relates to a vector containing the aforementioned DNA, and more particularly, to an expression vector. The expression vector contains a control sequence such as a promoter or terminator. An ordinary control sequence such as a promoter corresponding to the host used can be used for the control sequence. Prokaryotic cells such as Escherichia coli, eukaryotic microorganisms such as yeasts or filamentous fungi, or higher eukaryotic cells, namely cultured animal cells or cultured plant cells, can be used.

Although a sugar has been reported to first transfer to the 5 position of anthocyanidin during synthesis of rose anthocyanin, as a result of extensive research, an enzyme that transfers sugar to the 3 position has been unexpectedly demonstrated to be present also in roses. In addition, a gene that encodes that enzyme was able to be acquired. Synthesis of anthocyanin can be promoted by expressing this gene in roses or different species of plants. In addition, synthesis of anthocyanin can be inhibited by inhibiting expression thereof. In addition, by inhibiting synthesis of anthocyanin in roses, accumulation of anthocyanidin, and particularly cyanidin, can be promoted, thereby making it possible to also promote accumulation of rosacyanin.

In addition, a gene encoding an enzyme that transfers a sugar to the 3 position of anthocyanidin was clearly demonstrated to be involved in color changes following blooming of rose flowers. On the basis of the expression profile of this gene, the promoter region of this gene is thought to have the function of specifically expressing the gene after blooming. Roses in which flower color changes after blooming can be produced by molecular breeding using this type of anthocyanin synthesis gene involved in changes in flower color after blooming (containing an expression regulatory region such as a promoter of that gene).

### EXAMPLES

The following provides a detailed description of the present invention according to the examples indicated below. Unless specifically stated otherwise, molecular biology techniques were performed in accordance with Molecular Cloning (Cold Spring Harbor Laboratory Press, 2001) and Plant Physiol. (2003) 132, 1652-1663.

### Example 1 - Construction of Rose cDNA Library and Acquisition of Other 3GT cDNA Candidates

An enzyme (A3GT) gene that transfers a sugar to the 3 position of anthocyanidin contained in the petals of plants can be obtained by screening a cDNA library thereof with A3GT genes of different species. For example, gentian A3GT gene was able to be obtained by using snapdragon A3GT gene as a probe (Plant Cell Physiol. (1996) 37, 711-716). A cDNA library was constructed using RNA extracted from colored leaves (in which anthocyanin was accumulated) of roses (Rosa hybrida cultivar Samba) according to a known method (for example, Plant Cell Physiol. (1995) 36, 1023-1031).

Library construction was carried out using the λZAPII Directional cDNA Library Synthesis Kit (Stratagene Corp.) in accordance with the recommended method by the manufacturer. A cDNA library consisting of about 5,500,000 plaque forming units (pfu) was obtained. Portions corresponding to the sequence of the N-terminal sequences of two types of genes consisting of perilla-derived A3GT gene (Plant Mol. Biol. (1997) 35, 915-927) and gentian-derived A3GT gene (Plant Cell Physiol. (1996) 37, 711-716) were used as probes for library screening. A pair of oligonucleotide primers each as indicated below were synthesized to amplify the N-terminal sequence of each GT.
Perilla A3GT
SEQ ID NO. 5: 5'-CAC ATT GGC GTT CTA GCA TTC-3'
SEQ ID NO. 6: 5'-GCC TTC GGG GGT CCC GTC CCA-3'
Gentian A3GT
SEQ ID NO. 7: 5'-CAT GTT GCA GTG CTT GCA TTT-3'
SEQ ID NO. 8: 5'-TCC CTC CGG CGA GCC ATC CCA-3'

The probes were labeled by PCR using the Non-Radioisotope DIG-Nucleic Acid Detection System (Roche Diagnostics K.K.) in accordance with the conditions recommended by the manufacturer. At that time, 1 ng of plasmid containing each cDNA were used as templates, 100 ng of oligonucleotide specific to each gene as described above were used as primers, and PCR was carried out for 35 cycles with each cycle consisting of reacting for 1 minute at 94°C, 1 minute at 55°C and 1 minute at 72°C. The aforementioned cDNA library (approx. 300,000 pfu for each probe) was screened by using the PCR amplification products of each gene as hybridization probes.

The screening was carried out using the Non-Radioisotope DIG-Nucleic Acid Detection System (Roche Diagnostics K.K.) according to the method recommended by the manufacturer. Hybridization was carried out overnight at 37°C in 5xSSC containing 30% formaldehyde followed by washing the filter for 30 minutes at 55°C using 5xSSC containing 1% SDS. Secondary screening was further carried out on those clones for which a signal was detected in primary screening, and ultimately one kind of cDNA (pSPB1123) was obtained.

The resulting nucleotide sequence is shown in SEQ ID NO. 1, and the corresponding amino acid sequence is shown in SEQ ID NO. 2. When this amino acid sequence was compared with a known 3GT amino acid sequence, the amino acid sequence of the enzyme of the present invention demonstrated sequence identity of 42% with respect to perilla A3GT, sequence identity of 43% with respect to gentian A3GT and sequence identity of 44% with respect to grape A3GT (J. Biol. Chem. (1998) 273, 9224-9233). On the other hand, homology with a previously reported rose anthocyanidin 5,3-glucosyltransferase was 22%. The enzyme of the present invention is therefore a novel enzyme on the basis of the above.

### Example 2 - Activity of Protein Encoded by Rose 3GT cDNA Candidate

In order to analyze the activity of a protein encoded by cDNA contained in pSPB1123, this cDNA was expressed in yeast cells. Yeast expression vector pYE22m (Plant Cell Physiol. (1996) 37, 711-716) was used to express the cDNA in yeast. A fragment obtained by digested pSPB1123 cDNA with EcoRI and KpnI was inserted into an EcoRI and KpnI recognition sequence of pYE22m to obtain a yeast expression vector. This vector was then inserted into yeast strain G1315 followed by measurement of activity of the protein encoded by pSPB1123 according to the method described on pp. 114-118 of *Shinseibutsugaku Jikken* (New Biological Experiment) Manual 3 (Kagaku-Dojin Publishing Co., Inc.).

The method for measuring activity and the conditions for measuring a product by HPLC were the same as the method and the like described in Plant Physiology (2003) 132, 1652-1663. Furthermore, anthocyanin (cyanidin, pelargonidin), cyanidin 3-glucoside (cyanidin 3G), cyanidin 3,5-diglucoside (cyanidin 3,5G), flavonol (quercetin, kaempferol) and flavone (apigenin, luteolin) were used as substrates for measurement of the activity.

As a result, protein encoded by cDNA contained in pSPB1123 had significant activity that added glucose to flavonol, and was observed to tend to prefer quercetin to kaempferol. The product was confirmed to be a 3-glycosylation product of flavonol based on cochromatography with an authentic sample. Although there also appeared to be slight activity to add a glucose to cyanidin as well, when compared with the case of using flavonol as a substrate, only a very small amount of cyanidin 3-glucoside was formed. In addition, glycosylation activity to flavones was not observed. Thus, the glucosyltransferase encoded by this cDNA was determined to be a flavonol 3GT rather than an anthocyanidin 3GT. In addition, even when screening was repeated on the same library using cDNA contained in this pSPB1123 as a probe, a gene of an enzyme that transfers a sugar to the 3 position of anthocyanidin was unable to be obtained, and rose A3GT was unable to be easily obtained.

### Example 3 - Construction of Rose Genomic Library

Genomic DNA was extracted from the petals of a rose (cultivar: Lavande) using Phytopure (Amersham plc), and a genomic library was constructed as indicated below using λBlueSTAR in accordance with the recommended method of the manufacturer.

First, after partially digesting the extracted genomic DNA with a restriction enzyme Sau3AI, a partial fill-in reaction was carried out in accordance with the method recommended by Novagene Inc. followed by fractionating by a sucrose density gradient centrifugation. DNA fragments ranging from 12 to 24 kb were recovered, and after ligating with λBlueSTAR vector digestedin advance with XhoI, packaging was carried out using the Gigapack Packaging Kit (Stratagene Corp.). A genomic library consisting of about 1,000,000 pfu was obtained.

### Example 4 - Screening of Rose A3GT Gene

A N-terminal sequence of the GTs disclosed previously, which is highly conserved only in A3GTs were amplified and the rose cDNA library described in Example 1 was screened by using the fragment as probes. The GTs used for the probes consisted of the same perilla A3GT gene (Plant Mol. Biol. (1997) 35, 915-927) and gentian A3GT gene (Plant Cell Physiol. (1996) 37, 711-716) of Example 1 as well as snapdragon A3GT gene (Plant J. (1991) 1, 37) and petunia A3GT (Plant Mol. Biol. (2002) 48, 401), for a total of four types. The perilla and gentian A3GT genes were the same as in Example, while the following respective pairs of oligonucleotides were synthesized for the snapdragon and petunia A3GT genes in order to amplify the DNA sequences corresponding to the N-terminal sequences of each GT.
Snapdragon A3GT
SEQ ID NO. 9: 5'-CAC ATT GGC GTG CTA GCG TTC-3'
SEQ ID NO. 10: 5'-GCC CTC TGG GGT GCC ATC CCA-3'
Petunia A3GT
SEQ ID NO. 11: 5'-CAC ATT GCA CTT CTT GCT TTC-3'
SEQ ID NO. 12: 5'-TGT TTC AGT GAC ACC ATC CCA-3'

A labeled PCR amplification product of each gene prepared in the same manner as Example 1 was used as a hybridization probe to screen the rose genomic library described in Example 3 (approx. 300,000 pfu for each probe).

Screening was carried out using the Non-Radioisotope DIG-Nucleic Acid Detection System (Roche Diagnostics K.K.) according to the method recommended by the manufacturer. Hybridization was carried out overnight at 37°C in 5xSSC containing 30% formaldehyde followed by washing the filter for 30 minutes at 55°C using 5xSSC containing 1% SDS. Secondary screening was further carried out for those clones for which a signal was detected in primary screening, and ultimately 14 positive clones were obtained from those clones in which gentian A3GT gene was used as a probe. There were no positive clones obtained in the case of using any of the other A3GT genes than gentian's.

### Example 5 - Analysis of Obtained A3GT Candidate Genes

Plasmid DNA were recovered from the positive clones acquired in Example 2 using the λBlueSTAR Library Synthesis Kit in accordance with the method recommended by the manufacturer. In order to determine which parts of the cloned sequence 3GT gene is present, the obtained plasmid DNA was digested with restriction enzymes XhoI, SalI, HindIII and XbaI (sigle digestion) or using both EcoRI and BamHI followed by carrying out southern hybridization using the same probes derived from gentian A3GT gene used in Examples 1 and 4.

Southern hybridization was carried out by electrophoresis of each restriction enzyme-digested DNA in 8% agarose gel followed by using the aforementioned Non-Radioisotope DIG-Nucleic Acid Detection System DNA Labeling and Detection Kit in accordance with the method recommended by the manufacturer. Those fragments of the resulting genomic clones that demonstrated a strong signal in a southern hybridization were sub-cloned to a pBluescript II SK vector followed by analysis of their nucleotide sequences. Sequencing was carried out using the DNA Sequencer Model 3100 (Applied Biosystems Inc.) according to the primer walking method using synthesized oligonucleotide primers.

As a result of comparing the amino acid sequence predicted from the resulting sequence with those of known A3GT of other plants, a sequence demonstrating high identity with A3GT of other plants was determined to be present in an approximately 3.5 kg fragment resulting from cleavage of clone #16 with HindIII (designated as pSFL333). Identity between the amino acid sequence of the GT present in the sequence of this pSFL333 and the amino acid sequences of other A3GT was 46% with respect to gentian A3GT, 46% with respect to perilla A3GT, and 57% with respect to grape A3GT. In addition, identity at the amino acid level with flavonol 3GT encoded by pSFL1123 described in Example 1 was 42%. On the other hand, identity with previously reported rose anthocyanidin 5,3-glucosyltransferase was 21%.

The entire sequence of the pSFL333 cDNA is shown in SEQ ID NO. 3, while the corresponding amino acid sequence is shown in SEQ ID NO. 4. In addition, an intron was also determined to be present in this rose 3GT candidate gene at the location corresponding to the intron of Arabidopsis thaliana A3GT (UGT78D1). Although there were other genomic clones obtained from screening that had a sequence considered to be a GT gene besides clone #16, since they did not demonstrate high homology with known A3GT, subsequent analysis was focused only on pSFL333 cDNA.

### Example 6 - Construction of Escherichia coli

### Expression Construct of Resulting Anthocyanin 3GT Candidate Gene

Since an intron of about 180 bp was present in the resulted rose 3GT candidate gene about 500 bp downstream from the initiating methionine codon as shown in SEQ ID NO. 3, it was necessary to remove this intron when analyzing activity by expressing in E. coli.

Therefore, three fragments were prepared, consisting of (1) a fragment in which the region from the initiating methionine codon to just before the intron was amplified by PCR using Rose 3GT16-NcoI primer and Rose 3GT16-SpeI-1 primer, (2) a fragment in which the region from immediately after the intron to a KpnI recognition sequence present within the second exon was amplified by PCR using Rose 3GT-SpeI-3 primer and Rose 3GT-KpnI primer, and (3) a fragment in which the region from a KpnI recognition sequence present within the second exon to a BamHI recognition sequence present within the 3' untranslated region, and fragments (2) and (3) followed by fragment (1) were inserted into the NcoI and BglII recognition sequences of E. coli expression vector pQE61 (Qiagen, Inc.) in that order to construct an E. coli expression vector.

Furthermore, the nucleotide sequences of fragments (1) and (2) amplified by PCR were confirmed, and digested with NcoI and SpeI or SpeI and KpnI prior to insertion. The resulting E. coli expression vector was designated as pSFL344. The following indicates the sequences of the primers used to amplify fragments (1) and (2).

SEQ ID NO. 13: Rose 3GT16-NcoI 5'-GAC TCC ATG GCA
CCA GCA TCA AAT C-3'

SEQ ID NO. 14: Rose 3GT16-SpeI-1 5'-ACA CGT ACT AGT
TGT GTT CCT GAT G-3'

SEQ ID NO. 15: Rose 3GT16-SpeI-3 5'-AGT CAC TAG TAT
GGG TGG TCA CGA CGA TG-3'

SEQ ID NO. 16: Rose 3GT16-KpnI 5'-CGT TGA GGT ACC
TCT TGA ATT TGG-3'

### Example 7 - Measurement of Activity of Rose Anthocyanin 3GT

Expression constructs pSFL344 and pQE61 were introduced into E. coli strain JM109 (Toyobo Co., Ltd.) and pre-cultured overnight at 37°C in LB medium containing ampicillin at a final concentration of 20 µg/ml. A portion of the pre-culture broth was cultured to an A600 of 0.6 to 1.0 for about 2 hours in M9 medium containing 50 µg/ml of ampicillin and 0.5% casamino acids, brought to a volume of 10 ml, followed by the addition of IPTG (isopropyl-β-D-thiogalactopyranoside) to a final concentration of 0.1 mM and further culturing with shaking for 4 hours at 37°C. After centrifuging for 10 minutes at 3000 rpm and 4°C, the collected cells were suspended in 10 ml of buffer (30 mM Tris-HCl pH 7.5, 30 mM NaCl) followed by lysing the E. coli by ultrasonic treatment, carrying out centrifugal precipitation for 10 minutes at 15000 rpm and 4°C and using the resultant supernatant as a crude enzyme liquid for use in the following measurement of enzyme activity.

The reaction was carried out by mixing 30 µl of the crude enzyme liquid, 10 µl of 1 M Tris-HCl (pH 7.5), 5 µl of 5 mM UDP-glucose and 5 µl of 2 mM delphinidin or cyanidin, followed by holding for 15 minutes at 30°C. After stopping the reaction by adding an equal amount of 90% acetonitrile containing 0.1% TFA, the glycosylated products were detected in the same manner as the method described in Plant Physiol. (2003) 132, 1652-1663. In the reaction system using crude enzyme derived from E. coli expressing pSFL344, a specific product was detected that was not observed in the reaction system using crude enzyme derived from E. coli into which pQE60 is introduced. This product was identified as delphinidin 3-glucoside or cyanidin 3-glucoside based on cochromatography with a standard and agreement with the absorption spectrum of the standard.

On the basis of these results, a gene encoded by pSFL333 was confirmed to be that which encodes A3GT having activity that adds a sugar to the 3 position of anthocyanin.

### Example 8 - Inhibition of Expression of Flavonol 3GT Encoded by pSBP1123 cDNA in Roses

In order to assess effects in the case of inhibiting expression of the previously described rose anthocyanin 3GT (A3GT) and flavonol 3GT (F3GT) in rose petals, constructs were prepared for inhibiting expression of each 3GT and simultaneously inhibiting expression of both 3GT. First, with respect to flavonol 3GT encoded by pSPB1123, (1) a fragment extending from an SacI recognition sequence originating in the 5'-side vector to an SphI recognition sequence present in the vicinity of 900 bp from the 5'-terminal of the cDNA, and (2) a fragment extending from a BamHI recognition sequence originating in 5'-side vector sequence to an SphI recognition sequence inserted by PCR at a location about 750 bp from the 5'-terminal of the cDNA, were prepared.

The fragment of (2) was prepared by digesting with BamHI and SphI a DNA fragment amplified by PCR using the following primers by an ordinary methods (pSPB1123-SphI primer and reverse primer originating in the vector sequence).

SEQ ID NO. 17: pSPB1123-SphI 5'-AGA GTT GCA TGC AAC
CGC AGT T-3'

SEQ ID NO. 18: Reverse primer 5'-AAC AGC TAT GAC CAT
G-3'

A GUS gene located between an EI₂35S promoter and mas terminator of binary expression vector pSPB528 in the plant described in International Publication WO 2005/017147 was removed by cleaving with BamHI and SacI, a total of three DNA fragments consisting of this DNA fragment and the DNA fragments of (1) and (2) above were ligated to obtain pSFL224. Plasmid pSFL224 obtained in this manner, when introduced into a rose, constitutively transcribes double-stranded RNA originating in flavonol 3GT cDNA which pSPB1123 has, under the control of E1235S promoter. As a result, expression of rose flavonol 3GT is expected to be inhibited by RNAi.

### Example 9 - Inhibition of Expression of A3GT Encoded by pSFL333 cDNA in Roses

Anthocyanin 3GT encoded by pSFL333 was prepared by respectively digesting fragments consisting of (1) a fragment extending from an XhoI recognition sequence present in the vicinity of 50 bp from the 5'-terminal to an SpeI recognition sequence present in the vicinity of 835 bp from the 5'-terminal, and (2) a fragment extending from a BamHI recognition sequence originating in the 5'-side vector sequence to an SpeI recognition sequence present in the vicinity of about 630 bp from the 5'-terminal of the cDNA, with restriction enzymes, followed by electrophoresing and recovering the fragments from the gel. During preparation of fragment (1), SpeI-digestion was carried out by partial digestion by limiting the digestion time.

On the other hand, a plasmid in which a fragment extending from an HindIII recognition sequence to an EcoRI recognition sequence cut out from the vector pUE8 described in International Publication WO 2005/017147 (extending from an EI₂35S promoter to an nos terminator), is inserted to a HindIII recognition sequence and an EcoRI recognition sequence of pBINPLUS (Van Engelen et al., Transgenic Research 4 (1995) 288-290), was designated as pSPB176. A total of three fragments, consisting of a DNA fragment from which GUS gene had been removed by digesting with BamHI and SalI, and fragments (1) and (2) above, were then ligated to obtain pSFL223. The resulting plasmid pSFL223, when inserted into a rose, constitutively transcribes double-stranded RNA originating in A3GT cDNA contained in pSFL333 under the control of EI₂35S promoter. As a result, expression of rose A3GT is expected to be inhibited by RNAi.

### Example 10 - Inhibition of Expression of Both 3GT Encoded by pSPB1123 and pSFL333 cDNA in Roses

A construct was prepared for inhibiting both 3GT activities encoded by pSFL1123 and pSFL333. After digesting vector pSPB313 described in International Publication WO 2005/059141 with BamHI and SacI and removing F3H cDNA, this DNA fragment was ligated to fragments (1) and (2) described in Example 9, and the resultant plasmid was designated as pSFL225. A fragment extending from a promoter to a terminator obtained by cleaving pSFL225 with AscI was inserted into the AscI recognition sequence of pSFL223 prepared in Example 9 so as to have the same transcription orientation as the expression cassette of pSFL223, and the resulting construct was designated as pSFL226.

This pSFL226, when introduced into a rose, transcribes double-stranded RNA originating in pSFL1123 and pSFL333 cDNA under the control of EI₂35S promoter. As a result, expression of both of these rose flavonol and anthocyanidin 3GT is expected to be inhibited by RNAi.

### Example 11 - Analysis of Expression Mode of F3GT Gene Encoded by pSPB1123 in Roses

In order to analyze the expression mode of F3GT gene encoded by pSBP1123 in roses, RNA was extracted from the petals of rose cultivars consisting of Black Baccara, Rote Rose, Medeo, Madam Violet and Lavande during coloring (Plant Cell Physiol. (1995) 36, 1023-1031), followed by Northern hybridization in accordance with a known method (Plant Cell Physiol. (1995) 36, 1023-1031). Northern hybridization was carried out using a DIG Northern Kit (Amersham plc) according to the method recommended by the manufacturer. An RNA probe originating in the F3GT gene sequence labeled with DIG was used for the probe. Transcription products of the expected lengths were detected for all cultivars (Fig. 1).

### Example 12 - Analysis of Expression Mode of A3GT Gene Encoded by pSFL333 in Roses

In order to analyze the expression mode of A3GT gene encoded by pSFL333 in roses, RNA was extracted from the petals of roses followed by Northern hybridization. Northern hybridization was carried out using an RNA probe originating from an A3GT gene sequence labeled with DIG in the same manner as Example 11.

In rose cultivars such as Black Baccara, Rote Rose, Lavande, Medeo or Madam Violet, hardly any transcription products were detected even at the bud stage during which structural genes involved in anthocyanin synthesis in colored petals are normally expressed at a high level (Plant Cell Physiol. (1995) 36, 1023-1031) (Fig. 1). When the anthocyanin present in these cultivars of roses is analyzed, although cyanidin 3G is normally contained in extremely small amounts in petals immediately after blooming from buds, cyanidin 3,5G is present as nearly a single product.

On the other hand, in rose cultivar such as Ehigasa (Keihan Gardening Co., Ltd.) or Charleston that change color accompanying blooming, although similar to cultivar mentioned above, cyanidin 3,5G is present in large amounts in petals that are initially orange-yéllow, accompanying subsequent blooming, cyanidin 3G has been demonstrated to be accumulated in large amounts at the stage when the flowers become red (Fig. 2).

Therefore, RNA was extracted from the petals of a rose such as Ehigasa, which changes color accompanying blooming in this manner, from the stage of young bud to the stage at which aging starts after blooming (Fig. 2), and Northern hybridization was carried out using an RNA probe originating in A3GT gene sequence encoded by pSFL333 in the manner described above. As a result, this A3GT was determined be prominently expressed from the stage at which color change begins after blooming to the stage at which red coloring progresses, or in other words, during the stage at which cyanidin 3G accumulates (Fig. 3).

In addition, as a result of carrying out Northern hybridization using RNA probes originating in the respective gene sequences of rose chalcone synthase (CHS), dihydroflavonol reductase (DFR) and flavonoid 3'-hydroxylase (F3'H) that act as structural genes involved in the synthesis of anthocyanin in the same manner as described above, these genes were also determined to be prominently expressed during stage 4 in which this A3GT is expressed (Fig. 4). Each of these CHS, DFR and F3'H genes were expressed at higher levels in stage 4, during which cyanidin 3G is accumulated and petals become nearly completely red, rather than in stage 1, during which A5,3GT gene is expressed at a high level. On the basis of this finding, in cultivars such as Ehigasa that change cover after blooming, it is believed that the entire anthocyanin synthesis system is activated in the stage after blooming when clear aging begins (stage 4) rather than the stage during which anthocyanin synthesis is most active in ordinary roses (stage 1).

Moreover, on the basis of the above results, promoters and other transcription regulatory regions of this A3GT gene are predicted to have a function that specifically expresses this gene in petals that have progressed to the stage after blooming. Accordingly, it is thought to be possible to create a rose that changes color after blooming by ligating a coding region of a pigment synthesis gene to a promoter portion of A3GT shown in SEQ ID NO. 3 and inserting into a suitable rose. For example, if cDNA of pansy F3',5'H (International Publication WO 2004/020637) is ligated to the sequence of a promoter portion of A3GT and inserted into a suitable cultivar of rose, then it is thought that blue delphinidin will be synthesized accompanying blooming, making it possible to create a rose that changes to a blue color.

### Example 13 - Analysis of Rose Transformants Inserted with Construct Inhibiting F3GT Encoded by pSBP1123

pSFL224 was inserted into rose cultivar Lavande to suppress F3GT encoded by pSPB1123 as in Example 8. The flower colors of several transgenic lines became paler or whiter as compared with the host (Fig. 5). As a result of analyzing the flavonoid contents of the flowers of those lines that exhibited color changes in this manner by HPLC, the content of cyanidin 3,5G, which accounted for nearly 100% of the anthocyanin contained by the host petals, had decreased to roughly one-sixth that level in the transformants. In addition, flavonol content had increased to roughly twice that of the control. In addition, rosacyanin content remained unchanged.

DNA was extracted from the petals of the transformants that exhibited a change in petal color to confirm insertion of the construct down-regulating F3GT gene in the form of pSFL224.

Moreover, RNA was extracted from transformant #224-30, in which a change in petal color was observed, and from petals of the host Lavande cultivar, and Northern hybridization was carried out using RNA probes respectively originating in sequences consisting of the total length of F3GT gene, a 600 bp fragment on the 3'-side of F3GT gene, and rose A5,3GT gene (Fig. 6). In the case of using the full length of F3GT gene for the probe, the F3GT transcription product was higher in the transformants than in the host (Fig. 6). However, in the case of using a portion of endogenous F3GT gene that is not contained in pSFL224, namely a portion specific to endogenous F3GT gene, for the probe, although the transcription product was detected in the host, there were transcription products detected in the transformants. Here, the portion specific to endogenous F3GT gene used for the probe refers to a fragment which is not contained in pSFL224 and has about 600 bp on the 3'-terminal side from an SphI recognition sequence located at a location about 900 bp from the methionine starting codon of F3GT gene. Thus, although expression of endogenous F3GT gene is inhibited in transformants inserted with pSFL224, transcription products originating in the F3GT sequence of the inserted pSFL224 are thought to be present at a high level. In addition, transcription products were detected in the transformants that were of a different length than the inherently transcribed sequence.

On the other hand, the transcription product of A5,3GT was significantly reduced in transformants in which petal color became lighter. Expression of rose GAPDH gene, which was used as a positive control, was constant in all lines (Fig. 6).

On the basis of these results, in addition to expression of endogenous F3GT gene being suppressed by insertion of pSFL224 construct that inhibits expression of F3GT gene, expression of endogenous A5,3GT gene, which is said to be required for formation of anthocyanin, was also inhibited. As a result, the amount of anthocyanin in the pelals of the transformants decreased, which was thought to cause color to become lighter. In the case of inhibiting expression of a target gene by inserting an RNAi construct, although the transcription product of the RNAi construct itself typically decreases or disappears in coordination with the transcription product of the target gene, in this case, F3GT gene originating in the inserted pSFL224 is thought to have been expressed in excess even though expression of the target gene (rose endogenous F3GT gene) was inhibited.

### Example 14 - Analysis of Rose Transformants Inserted with Construct Inhibiting Expression of A3GT Encoded by pSFL333

pSFL223 was introduced into rose cultivar Lavande to inhibit expression of A3GT encoded by pSFL333 as in Example 9. Although about 30 transformants were obtained, none were observed to exhibit changes in flower color. In addition, although the pigments of five of the transformants were analyzed by HPLC, there were no differences between the contents of anthocyanin and rosacyanin contained in the petals immediately after blooming and the contents in the control.

In addition, as was described in Example 12, A3GT encoded by pSFL333 is virtually not expressed or only expressed at a low level at which transcription products are unable to be confirmed by Northern hybridization in the host Lavande rose (Fig. 1). Accordingly, although the presence or absence of transcription products of A3GT gene in the transformants was analyzed by Northern hybridization, no transcription products were detected in any of the transformants in the same manner as the control.

### INDUSTRIAL APPLICABILITY

As indicated in Example 13, rose F3GT gene encoded by pSFL1123 inhibits anthocyanin synthesis in roses, and can be used to produce roses that change color.

On the other hand, A3GT gene encoded by pSFL333 was determined to be specifically involved in anthocyanin synthesis in the stage in which color changes following blooming, instead of anthocyanin synthesis in the stage of the initial bud, in rose cultivars that change color such as Picture Parasol. On the basis of this finding, this A3GT gene, and particularly a promoter region thereof, can be used to produce flowers that change color accompanying blooming by genetic recombination.

## Claims

1. A nucleic acid encoding a protein originating in *Rosaceae Rosa* having activity that transfers a sugar to a 3-position hydroxyl group of a flavonoid.

2. The nucleic acid according to claim 1, wherein the flavonoid is anthocyanidin or flavonol.

3. A nucleic acid encoding a protein having an amino sequence indicated in SEQ ID NO. 2 or SEQ ID NO. 4, which has activity that transfers a sugar to a 3-position hydroxyl group of a flavonoid.

4. A nucleic acid encoding a protein having an amino acid sequence that has been modified by addition, deletion and/or substitution with other amino acids to one or a plurality of amino acids in an amino acid sequence indicated in SEQ ID NO. 2 or SEQ ID NO. 4, and having activity that transfers a sugar to a 3-position hydroxyl group of a flavonoid.

5. A nucleic acid encoding a protein having an amino acid sequence that has been modified by addition, deletion and/or substitution with other amino acids to one or several amino acids in an amino acid sequence indicated in SEQ ID NO. 2 or SEQ ID NO. 4, and having activity that transfers a sugar to a 3-position hydroxyl group of a flavonoid.

6. A nucleic acid encoding a protein having sequence identity of at least 60% with respect to an amino acid sequence indicated in SEQ ID NO. 2 or SEQ ID NO. 4, and having activity that transfers a sugar to a 3-position hydroxyl group of a flavonoid.

7. A nucleic acid encoding a protein having sequence identity of at least 80% with respect to an amino acid sequence indicated in SEQ ID NO. 2 or SEQ ID NO. 4, and having activity that transfers a sugar to a 3-position hydroxyl group of a flavonoid.

8. A nucleic acid encoding a protein having sequence identity of at least 90% with respect to an amino acid sequence indicated in SEQ ID NO. 2 or SEQ ID NO. 4, and having activity that transfers a sugar to a 3-position hydroxyl group of a flavonoid.

9. A nucleic acid encoding a protein having an amino acid sequence encoded by a nucleic acid that hybridizes under moderately stringent conditions with a nucleic acid having a nucleotide sequence of a coding region indicated in SEQ ID NO. 1 or SEQ ID NO. 3, and having activity that transfers a sugar to a 3-position hydroxyl group of a flavonoid.

10. A nucleic acid encoding a protein having an amino acid sequence encoded by a nucleic acid that hybridizes under high stringent conditions with a nucleic acid having a base sequence of a coding region indicated in SEQ ID NO. 1 or SEQ ID NO. 3, and having activity that transfers a sugar to a 3-position hydroxyl group of a flavonoid.

11. A nucleic acid according to claim 3, containing a nucleotide sequence of a coding region indicated in SEQ ID NO. 1 or SEQ ID NO. 3.

12. A nucleic acid containing a sequence complementary to the nucleic acid according to any of claims 1 to 11.

13. The nucleic acid according to any of claims 1 to 12, wherein the nucleic acid is DNA or RNA.

14. A vector containing the nucleic acid according to any of claims 1 to 13.

15. A host transformed by the vector according to claim 14.

16. A protein encoded by the nucleic acid according to any of claims 1 to 13.

17. A method of producing a protein having activity that transfers a sugar to a 3-position hydroxyl group of a flavonoid, comprising: culturing or growing the host according to claim 15, and harvesting the protein from the host.

18. A plant into which the nucleic acid according to any of claims 1 to 13 has been inserted, a progeny thereof having identical properties thereto, or tissue or vegetative growth body thereof.

19. A plant into which the nucleic acid according to any of claims 1 to 13 has been inserted and for which flower color has been altered, progeny thereof having identical properties thereto, or tissue or vegetative growth body thereof.

20. A plant according to claim 18 or 19, or a cut flower of a progeny thereof having identical properties thereto.

21. A method for producing a plant in which color changes as a result of blooming comprising the use of the gene described in SEQ ID NO. 3 or a portion thereof.

22. The method according to claim 21, wherein the plant referred to in claim 21 is a rose.

23. A plant that changes color after blooming produced according to the method according to claim 21 or 22, progeny thereof having identical properties thereto, or tissue or vegetative growth body thereof.

24. The plant according to claim 23, or a cut flower of a progeny thereof having identical properties thereto.


SEQUENCE LISTING
<110>International Flower Developments Pty. Ltd.
<120>Flavonoid glycosyl transferase and use thereof
<130>
<140>PCT/JP2007/053342
<141>2007-02-16
<150>JP2006-041280
<151>2006-02-17
<160>
<210>1
<211>1578
<212>DNA
<213>Rose
<220>
<221>
<223>Nucleotide sequecse of gene encoding rose flavonoid
glycosyl transferase
<400>1
gctggagctc tatcaatctc cccttaaagt cgttggtcga tcctcttcat ttcagttcat 60
ttctcacttg aaaggctgaa agtttgcaca atg agc cac aaa ctt gct agt tct 114
Met Ser His Lys Leu Ala Ser Ser
1               5
aat cct caa gcc cct gtg gca gtt ttt aat ttt cca ttc gcc tcc cat 162
Asn Pro Gln Ala Pro Val Ala Val Phe Asn Phe Pro Phe Ala Ser His
    10                  15                  20
ccc agc tct ctt ctc cga ttt ata cga acc att tcg gcc gtt gcc ccg 210
Pro Ser Ser Leu Leu Arg Phe Ile Arg Thr Ile Ser Ala Val Ala Pro
25                  30                  35                  40
gac att aag ttc acc ttc ttc agc act tct aga aca aat agc tcc ctc 258
Asp Ile Lys Phe Thr Phe Phe Ser Thr Ser Arg Thr Asn Ser Ser Leu
                45                  50                  55
ttc tcg ggt tcc aac aac aag ggc ttg gat aat gta aag cct tac aat 306
Phe Ser Gly Ser Asn Asn Lys Gly Leu Asp Asn Val Lys Pro Tyr Asn
            60                  65                  70
gta tgg gat ggc ttg ccg gag ggt tac gtg ccc ccc tct gga cct ccg 354
Val Trp Asp Gly Leu Pro Glu Gly Tyr Val Pro Pro Ser Gly Pro Pro
        75                  80                  85

ctg gag caa att ggg ttg ttc ctt gac cag gca cct caa agc ttt gag 402
Leu Glu Gln Ile Gly Leu Phe Leu Asp Gln Ala Pro Gln Ser Phe Glu
    90                  95                  100
agg gca ctc aaa gaa gtg gag gcc gaa aca gga cac aag ttc ggc tgc 450
Arg Ala Leu Lys Glu Val Glu Ala Glu Thr Gly His Lys Phe Gly Cys
105                 110                 115                 120
ttg att tcg gat gca ttt ctc tgg ttt tcc ggg gac att gcc cag aaa 498
Leu Ile Ser Asp Ala Phe Leu Trp Phe Ser Gly Asp Ile Ala Gln Lys
                125                 130                 135
atg aac gtc cct tgg gtg act aca tgg agt gga cca cgc ccg ctg ctt 546
Met Asn Val Pro Trp Val Thr Thr Trp Ser Gly Pro Arg Pro Leu Leu
            140                 145                 150
gtt cat ctg gag act gat atg atc aga gaa aaa gtg gga gct cct ggg 594
Val His Leu Glu Thr Asp Met Ile Arg Glu Lys Val Gly Ala Pro Gly
        155                 160                 165
caa gaa gac aag acc ctg gac ttc ctt cca gga ttc tca aat gag ttc 642
Gln Glu Asp Lys Thr Leu Asp Phe Leu Pro Gly Phe Ser Asn Glu Phe
    170                 175                 180
cta gca tct gac tta cca aag gaa gtt gta ttt gga aac ata gag tcg 690
Leu Ala Ser Asp Leu Pro Lys Glu Val Val Phe Gly Asn Ile Glu Ser
185                 190                 195                 200
cca ctt gca aat atg ttg tat aaa atg gga cag aag ctg cca caa gca 738
Pro Leu Ala Asn Met Leu Tyr Lys Met Gly Gln Lys Leu Pro Gln Ala
                205                 210                 215
act gcg gtt gca gtc aac tct ttt gaa aca atg gac ttg aaa gtt tcc 786
Thr Ala Val Ala Val Asn Ser Phe Glu Thr Met Asp Leu Lys Val Ser
            220                 225                 230
gag gag ctg aag aaa aga ctc aag aag ttg ctt ctt gtt ggg cca cta 834
Glu Glu Leu Lys Lys Arg Leu Lys Lys Leu Leu Leu Val Gly Pro Leu
        235                 240                 245
cat cta gtc cgg cca gta cca tca gca caa tca gat gat gac gag gag 882
His Leu Val Arg Pro Val Pro Ser Ala Gln Ser Asp Asp Asp Glu Glu
    250                 255                 260
gag aag gat gca tgc tta ccg tgg ctg gac aac cac aag cct gca tca 930
Glu Lys Asp Ala Cys Leu Pro Trp Leu Asp Asn His Lys Pro Ala Ser
265                 270                 275                 280
gta gca tac atc agc ttt gga agt gtc gga gca ctg ccc cca atg gag 978

Val Ala Tyr Ile Ser Phe Gly Ser Val Gly Ala Leu Pro Pro Met Glu
                285                 290                 295
gta gca gca tta gcc gag gca tta gag gaa ggg gga ttc cca ttt ctt 1026
Val Ala Ala Leu Ala Glu Ala Leu Glu Glu Gly Gly Phe Pro Phe Leu
            300                 305                 310
tgg tca ttt agg ggg aac cta gag gac ttt ccc aaa gga ttc att gaa 1074
Trp Ser Phe Arg Gly Asn Leu Glu Asp Phe Pro Lys Gly Phe Ile Glu
        315                 320                 325
aga aca tct act gga aga gtt gtt ccc tgg gtg aac cag gtg caa atc 1122
Arg Thr Ser Thr Gly Arg Val Val Pro Trp Val Asn Gln Val Gln Ile
    330                 335                 340
cta aat cat ccg tcg ata ggg gtg ttt gtt aca cat ggc ggt tgg aag 1170
Leu Asn His Pro Ser Ile Gly Val Phe Val Thr His Gly Gly Trp Lys
345                 350                 355                 360
tca gtt ttg gag agt gta act tgt ggt gtg cct atg att ggg agg cct 1218
Ser Val Leu Glu Ser Val Thr Cys Gly Val Pro Met Ile Gly Arg Pro
                365                 370                 375
cat ttt gct gat caa act ctg aat atg cgg agc gta gaa gtt gta tgg 1266
His Phe Ala Asp Gln Thr Leu Asn Met Arg Ser Val Glu Val Val Trp
            380                 385                 390
aag atc ggt atg aga atc gag ggt ggc gtt ttc act aaa tct gga gca 1314
Lys Ile Gly Met Arg Ile Glu Gly Gly Val Phe Thr Lys Ser Gly Ala
        395                 400                 405
atc aag gta ttg gaa caa gct cta tcg ctt gag caa gga aaa gaa atg 1362
Ile Lys Val Leu Glu Gln Ala Leu Ser Leu Glu Gln Gly Lys Glu Met
    410                 415                 420
aga cag aga gtt gga gtc ctt aaa cag ctt gct caa gag act gtt gga 1410
Arg Gln Arg Val Gly Val Leu Lys Gln Leu Ala Gln Glu Thr Val Gly
425                 430                 435                 440
ccc aat ggg agt tca gct caa gac ttg aaa gtc tgg tagagatcat 1456
Pro Asn Gly Ser Ser Ala Gln Asp Leu Lys Val Trp
                445                 450
caaatcatga ttgcgaagtt taattactgt tggtctgaaa tcatgtcatg atggccaatg 1516
catctgagtt taaatcatct atcaataatt tctgttctgg tcttaaaaaa aaaaaaaaaa 1576
aa 1578
<210>2
<211>452
<212>PRT

<213>Rose
<220>
<221>
<223>Amino acid sequecse of rose flavonoid glycosyl transferase
<400>2
Met Ser His Lys Leu Ala Ser Ser Asn Pro Gln Ala Pro Val Ala Val
1               5                   10                  15
Phe Asn Phe Pro Phe Ala Ser His Pro Ser Ser Leu Leu Arg Phe Ile
            20                  25                  30
Arg Thr Ile Ser Ala Val Ala Pro Asp Ile Lys Phe Thr Phe Phe Ser
        35                  40                  45
Thr Ser Arg Thr Asn Ser Ser Leu Phe Ser Gly Ser Asn Asn Lys Gly
    50                  55                  60
Leu Asp Asn Val Lys Pro Tyr Asn Val Trp Asp Gly Leu Pro Glu Gly
65                  70                  75                  80
Tyr Val Pro Pro Ser Gly Pro Pro Leu Glu Gln Ile Gly Leu Phe Leu
                85                  90                  95
Asp Gln Ala Pro Gln Ser Phe Glu Arg Ala Leu Lys Glu Val Glu Ala
            100                 105                 110
Glu Thr Gly His Lys Phe Gly Cys Leu Ile Ser Asp Ala Phe Leu Trp
        115                 120                 125
Phe Ser Gly Asp Ile Ala Gln Lys Met Asn Val Pro Trp Val Thr Thr
    130                 135                 140
Trp Ser Gly Pro Arg Pro Leu Leu Val His Leu Glu Thr Asp Met Ile
145                 150                 155                 160
Arg Glu Lys Val Gly Ala Pro Gly Gln Glu Asp Lys Thr Leu Asp Phe
                165                 170                 175
Leu Pro Gly Phe Ser Asn Glu Phe Leu Ala Ser Asp Leu Pro Lys Glu
            180                 185                 190
Val Val Phe Gly Asn Ile Glu Ser Pro Leu Ala Asn Met Leu Tyr Lys
        195                 200                 205
Met Gly Gln Lys Leu Pro Gln Ala Thr Ala Val Ala Val Asn Ser Phe
    210                 215                 220
Glu Thr Met Asp Leu Lys Val Ser Glu Glu Leu Lys Lys Arg Leu Lys
225                 230                 235                 240
Lys Leu Leu Leu Val Gly Pro Leu His Leu Val Arg Pro Val Pro Ser
                245                 250                 255

Ala Gln Ser Asp Asp Asp Glu Glu Glu Lys Asp Ala Cys Leu Pro Trp
            260                 265                 270
Leu Asp Asn His Lys Pro Ala Ser Val Ala Tyr Ile Ser Phe Gly Ser
        275                 280                 285
Val Gly Ala Leu Pro Pro Met Glu Val Ala Ala Leu Ala Glu Ala Leu
    290                 295                 300
Glu Glu Gly Gly Phe Pro Phe Leu Trp Ser Phe Arg Gly Asn Leu Glu
305                 310                 315                 320
Asp Phe Pro Lys Gly Phe Ile Glu Arg Thr Ser Thr Gly Arg Val Val
                325                 330                 335
Pro Trp Val Asn Gln Val Gln Ile Leu Asn His Pro Ser Ile Gly Val
            340                 345                 350
Phe Val Thr His Gly Gly Trp Lys Ser Val Leu Glu Ser Val Thr Cys
        355                 360                 365
Gly Val Pro Met Ile Gly Arg Pro His Phe Ala Asp Gln Thr Leu Asn
    370                 375                 380
Met Arg Ser Val Glu Val Val Trp Lys Ile Gly Met Arg Ile Glu Gly
385                 390                 395                 400
Gly Val Phe Thr Lys Ser Gly Ala Ile Lys Val Leu Glu Gln Ala Leu
                405                 410                 415
Ser Leu Glu Gln Gly Lys Glu Met Arg Gln Arg Val Gly Val Leu Lys
            420                 425                 430
Gln Leu Ala Gln Glu Thr Val Gly Pro Asn Gly Ser Ser Ala Gln Asp
        435                 440                 445
Leu Lys Val Trp
    450
<210>3
<211>3507
<212>DNA
<213>Rose
<220>
<221>
<223>Nucleotide sequecse of gene encoding rose flavonoid
glycosyl transferase
<400>3
aagcttgacc catgacgacg acgacggcaa tttcacttca aaaaaaaaag aaaaaaaaga 60
aagaaaggaa aacagagaaa aagaaaaggt cgccgatctc tttctctgct tgtataaagt 120
gggtgatgga ctttaggagt tggggggcga ctaaattagc agacttgcgg tggcgatcaa 180

tttttagaga gataaacaac ccgcgccagc cgtgaggaga tttatgagtg atagagagaa 240
aatagaaaca aagtaaaggt cgcgtttctt ttctaatttc tgcagcaacc caaaaagaat 300
aacacatgag atattctttc attttctttc ttttagtgga cttactgacc cagctttcag 360
gcccatcttg ggcctattgg gctttttctt gaaatgagtc aacctgtctt aaaccaatct 420
ggctcgtggg cctggccggg ggttaccgcg tgctcaagtg ttactgtaga agcctgccgg 480
tctcctccta gaaataaaac ttttgtttag tggttggctt gtgacaacca ccgttgttac 540
tatactactt tggttctata aatacagacc aaagccacat ggaaaaaata gaattgcgac 600
cactgaagta ggtccatata tatatatccc tcacgtaact tgagctttac gtagtttcta 660
tacccaaacc ttgacttc atg gca cca gca tca aat cag gtg ggt ggt cat 711
Met Ala Pro Ala Ser Asn Gln Val Gly Gly His
1               5                   10
gtg gcg gtg cta gcc ttt ccc ttc tcg act cac gcc gcc ccg ctg ttg 759
Val Ala Val Leu Ala Phe Pro Phe Ser Thr His Ala Ala Pro Leu Leu
            15                  20                  25
aac atc gtc tgc cgc ctc gcc gcc gcc gcc cca aac acc ctc ttc tca 807
Asn Ile Val Cys Arg Leu Ala Ala Ala Ala Pro Asn Thr Leu Phe Ser
        30                  35                  40
ttc ttc aac acc aag caa tcc aat agc tcg atc cta gca agc aac acg 855
Phe Phe Asn Thr Lys Gln Ser Asn Ser Ser Ile Leu Ala Ser Asn Thr
    45                  50                  55
tca tcc ata cta cgt aac agt aac gta agg gtg tgt gag gtg gcg gat 903
Ser Ser Ile Leu Arg Asn Ser Asn Val Arg Val Cys Glu Val Ala Asp
60                  65                  70                  75
ggt gtg ccg gag ggg tat gtt ttt gtg ggt aag cct cag gag gac ata 951
Gly Val Pro Glu Gly Tyr Val Phe Val Gly Lys Pro Gln Glu Asp Ile
                80                  85                  90
gag ttg ttc atg aag gct gcc ccg gac aac ttc agg cgg tgc tta gag 999
Glu Leu Phe Met Lys Ala Ala Pro Asp Asn Phe Arg Arg Cys Leu Glu
            95                  100                 105
gcg tcc gtg gcg gag acc ggg agg gag gtc agc tgc ttg gtc act gac 1047
Ala Ser Val Ala Glu Thr Gly Arg Glu Val Ser Cys Leu Val Thr Asp
        110                 115                 120
gct ttc ttt tgg ttc ggg gct cac atg gcc gat gac ttg gga gga ctg 1095
Ala Phe Phe Trp Phe Gly Ala His Met Ala Asp Asp Leu Gly Gly Leu
    125                 130                 135
cct tgg gta ccg ttt tgg acc gcc ggg cca gct tca ctc tcc gct cat 1143
Pro Trp Val Pro Phe Trp Thr Ala Gly Pro Ala Ser Leu Ser Ala His
140                 145                 150                 155

gta cac acc gat ctc atc agg aac aca act agtacgtgta tacatatatt 1193
Val His Thr Asp Leu Ile Arg Asn Thr Thr
                160                 165
atgattcatc aatatcatat ataccgaatt attaatgctt tatttaatta gtctcgtcat 1253
gtgtgtacca aaacaagcag acacaagtac acatacatat ctattgacac caaaatggtt 1313
atattactag ctgacttgtc tcgatcattc atatgttcaa ataaaca ggt atg ggt 1369
Ser Met Gly
ggt cac gat gga aaa gaa acc atc act gca gtc act gca gga atg tcc 1417
Gly His Asp Gly Lys Glu Thr Ile Thr Ala Val Thr Ala Gly Met Ser
    170                 175                 180
aaa gta cga ccc cag gat ttg cca gag gga atc atc ttt gga aag ttg 1465
Lys Val Arg Pro Gln Asp Leu Pro Glu Gly Ile Ile Phe Gly Lys Leu
185                 190                 195                 200
gac tcc ctc ttt tca cgg atg cta cac cag atg gga cta atg cta cca 1513
Asp Ser Leu Phe Ser Arg Met Leu His Gln Met Gly Leu Met Leu Pro
                205                 210                 215
ctt gca aca gca gtt ttc atc aac tcc ttc gaa gaa cta gac cct gtg 1561
Leu Ala Thr Ala Val Phe Ile Asn Ser Phe Glu Glu Leu Asp Pro Val
            220                 225                 230
atc aca aat gat ttg aag tcc aaa ttc aag agg tac ctc aac gtg gga 1609
Ile Thr Asn Asp Leu Lys Ser Lys Phe Lys Arg Tyr Leu Asn Val Gly
        235                 240                 245
ccc ttc gac cta cta gag tca cca gca cca gca gcc acc acc aca ctg 1657
Pro Phe Asp Leu Leu Glu Ser Pro Ala Pro Ala Ala Thr Thr Thr Leu
    250                 255                 260
cag act ggg gac gtt gtt gtc gga gat ggc tgc tta tcg tgg ctt gac 1705
Gln Thr Gly Asp Val Val Val Gly Asp Gly Cys Leu Ser Trp Leu Asp
265                 270                 275                 280
aaa cag aag gcg gcg tct gta gtg tat gtg agt ttt gga tca gta aca 1753
Lys Gln Lys Ala Ala Ser Val Val Tyr Val Ser Phe Gly Ser Val Thr
                285                 290                 295
aga cca tcg ccg gag gag ctt atg gcg cta gcc gag gcg ttg gaa gcc 1801
Arg Pro Ser Pro Glu Glu Leu Met Ala Leu Ala Glu Ala Leu Glu Ala
            300                 305                 310
agt agg gtt cca ttc ttg tgg tct ctt cgg aac aat ttg atg act cct 1849
Ser Arg Val Pro Phe Leu Trp Ser Leu Arg Asn Asn Leu Met Thr Pro
        315                 320                 325

aag cta gac gag ttc ata agc aaa gca gaa tta aac ggg atg gtg gtg 1897
Lys Leu Asp Glu Phe Ile Ser Lys Ala Glu Leu Asn Gly Met Val Val
    330                 335                 340
cct tgg gtg cca caa cca cag gtc cta gca cat ggt tca gtt gga gca 1945
Pro Trp Val Pro Gln Pro Gln Val Leu Ala His Gly Ser Val Gly Ala
345                 350                 355                 360
ttc gta aca cat tgt ggt tgg aac tcg gtg ctt gag agc cta gca ggt 1993
Phe Val Thr His Cys Gly Trp Asn Ser Val Leu Glu Ser Leu Ala Gly
                365                 370                 375
gga gtg ccc atg atc tgc agg cct ttc ttc ggc gat cag aaa ctt aac 2041
Gly Val Pro Met Ile Cys Arg Pro Phe Phe Gly Asp Gln Lys Leu Asn
            380                 385                 390
gcg agg atg gta gag gat gag tgg aag att ggt ctc aaa ttg gag ggt 2089
Ala Arg Met Val Glu Asp Glu Trp Lys Ile Gly Leu Lys Leu Glu Gly
        395                 400                 405
ggg gtt ttc acc aag aat ggg atg ctt aag agt ttg gac ata cta ctg 2137
Gly Val Phe Thr Lys Asn Gly Met Leu Lys Ser Leu Asp Ile Leu Leu
    410                 415                 420
tcg caa aag aag ggg aac ata atg aga gac acg atc aac aca ttc aaa 2185
Ser Gln Lys Lys Gly Asn Ile Met Arg Asp Thr Ile Asn Thr Phe Lys
425                 430                 435                 440
caa ctc gca caa cag gcc gta gaa cca aaa ggg agc tcc act agg aac 2233
Gln Leu Ala Gln Gln Ala Val Glu Pro Lys Gly Ser Ser Thr Arg Asn
                445                 450                 455
ttt gaa tca ttg ttg gaa gtg ata tct aca gcc aat taagtataac 2279
Phe Glu Ser Leu Leu Glu Val Ile Ser Thr Ala Asn
            460                 465
cacaaatata ttccagaaga acaaagaact actaagggtt acagttgaat gaaagtgctt 2339
cgacttgtct gaataaaatt ctacttatac ttctaatcat ccttcataga aaagccatag 2399
ttcttcatac aaccttgctc aaatatgcta gtcgtcttgt tgatagtaca aaatatcatc 2459
gtttacactg aaactaccgt atccaacaaa aatcaagcaa actattcact aacatagagg 2519
actatatata ttggacccaa tcgaattgaa tctgtgggag gaaagaaaac caaagcattg 2579
agttgcccca ataagagggg caagatattt ctgcacaacc cataaaggta agtatacatt 2639
acactaagaa gaattatcaa tattaatagt catttaaaca gattgaatcc catctagcaa 2699
gtacatatag atcaatctgg atccagcaaa acccgtacaa atgactcgac atacccctct 2759
ccctaaatca gcacctaaat actctacagc catccaatag acatataggc agtgcagcca 2819
gcgggctacg ggtgcccttg tgcttttact aattttgacc aagaaaattc tggaaacaaa 2879
agggaatcta attagggaaa aaatgctgta tttgcattga cagattccaa caactgaatc 2939

taggtaccgc actgcatctc catcaatcac ccatgaattt agctcccaca tgaatattag 2999
ccatctagga atcaaccatt tcatcttgta tctcattggg ggcaacgagc ccaggaatag 3059
aaagcatccg ttgccgttct ctttctcttt gtacagcagc ctcctctgca taaagatctt 3119
tgttgtcctg caacagagaa agcaaacctc taattatact tgactaaaat cacaatgtct 3179
gacaaagtaa cagtgactta tcccaagcat aaaacagatt caagaaaaag aacatcattt 3239
gatactggta gtatcctacc tgtgctgaaa attcttttga ttgcactaga aagtctcgga 3299
tgtgatcctt aaatgtggcc aagtcattct tggactcgaa tagtccattc acaaattgag 3359
tgacctgagg aaaaaaaaaa aaacaaataa ataaaaccct tcagatgagt ggtattctta 3419
tggaattagt attctgggta caaacaaaat aatattagaa atatacctct atggaagtca 3479
tgttggggaa ggaatcactc aaaagctt 3507
<210>4
<211>468
<212>PRT
<213>Rose
<220>
<221>
<223>Amino acid sequecse of rose flavonoid glycosyl transferase
<400>4
Met Ala Pro Ala Ser Asn Gln Val Gly Gly His Val Ala Val Leu Ala
1               5                   10                  15
Phe Pro Phe Ser Thr His Ala Ala Pro Leu Leu Asn Ile Val Cys Arg
            20                  25                  30
Leu Ala Ala Ala Ala Pro Asn Thr Leu Phe Ser Phe Phe Asn Thr Lys
        35                  40                  45
Gln Ser Asn Ser Ser Ile Leu Ala Ser Asn Thr Ser Ser Ile Leu Arg
    50                  55                  60
Asn Ser Asn Val Arg Val Cys Glu Val Ala Asp Gly Val Pro Glu Gly
65                  70                  75                  80
Tyr Val Phe Val Gly Lys Pro Gln Glu Asp Ile Glu Leu Phe Met Lys
                85                  90                  95
Ala Ala Pro Asp Asn Phe Arg Arg Cys Leu Glu Ala Ser Val Ala Glu
            100                 105                 110
Thr Gly Arg Glu Val Ser Cys Leu Val Thr Asp Ala Phe Phe Trp Phe
        115                 120                 125
Gly Ala His Met Ala Asp Asp Leu Gly Gly Leu Pro Trp Val Pro Phe
    130                 135                 140
Trp Thr Ala Gly Pro Ala Ser Leu Ser Ala His Val His Thr Asp Leu
145                 150                 155                 160

Ile Arg Asn Thr Thr Ser Met Gly Gly His Asp Gly Lys Glu Thr Ile
                165                 170                 175
Thr Ala Val Thr Ala Gly Met Ser Lys Val Arg Pro Gln Asp Leu Pro
            180                 185                 190
Glu Gly Ile Ile Phe Gly Lys Leu Asp Ser Leu Phe Ser Arg Met Leu
        195                 200                 205
His Gln Met Gly Leu Met Leu Pro Leu Ala Thr Ala Val Phe Ile Asn
    210                 215                 220
Ser Phe Glu Glu Leu Asp Pro Val Ile Thr Asn Asp Leu Lys Ser Lys
225                 230                 235                 240
Phe Lys Arg Tyr Leu Asn Val Gly Pro Phe Asp Leu Leu Glu Ser Pro
                245                 250                 255
Ala Pro Ala Ala Thr Thr Thr Leu Gln Thr Gly Asp Val Val Val Gly
            260                 265                 270
Asp Gly Cys Leu Ser Trp Leu Asp Lys Gln Lys Ala Ala Ser Val Val
        275                 280                 285
Tyr Val Ser Phe Gly Ser Val Thr Arg Pro Ser Pro Glu Glu Leu Met
    290                 295                 300
Ala Leu Ala Glu Ala Leu Glu Ala Ser Arg Val Pro Phe Leu Trp Ser
305                 310                 315                 320
Leu Arg Asn Asn Leu Met Thr Pro Lys Leu Asp Glu Phe Ile Ser Lys
                325                 330                 335
Ala Glu Leu Asn Gly Met Val Val Pro Trp Val Pro Gln Pro Gln Val
            340                 345                 350
Leu Ala His Gly Ser Val Gly Ala Phe Val Thr His Cys Gly Trp Asn
        355                 360                 365
Ser Val Leu Glu Ser Leu Ala Gly Gly Val Pro Met Ile Cys Arg Pro
    370                 375                 380
Phe Phe Gly Asp Gln Lys Leu Asn Ala Arg Met Val Glu Asp Glu Trp
385                 390                 395                 400
Lys Ile Gly Leu Lys Leu Glu Gly Gly Val Phe Thr Lys Asn Gly Met
                405                 410                 415
Leu Lys Ser Leu Asp Ile Leu Leu Ser Gln Lys Lys Gly Asn Ile Met
            420                 425                 430
Arg Asp Thr Ile Asn Thr Phe Lys Gln Leu Ala Gln Gln Ala Val Glu
        435                 440                 445
Pro Lys Gly Ser Ser Thr Arg Asn Phe Glu Ser Leu Leu Glu Val Ile
    450                 455                 460

Ser Thr Ala Asn
465
<210>5
<211>21
<212>DNA
<213>Artificial Sequence
<220>
<221>
<223>Probe
<400>5
cacattggcg ttctagcatt c
<210>6
<211>21
<212>DNA
<213>Artificial Sequence
<220>
<221>
<223>Probe
<400>6
gccttcgggg gtcccgtccc a
<210>7
<211>21
<212>DNA
<213>Artificial Sequence
<220>
<221>
<223>Probe
<400>7
catgttgcag tgcttgcatt t
<210>8
<211>21
<212>DNA
<213>Artificial Sequence
<220>
<221>

<223>Probe
<400>8
tccctccggc gagccatccc a
<210>9
<211>21
<212>DNA
<213>Artificial Sequence
<220>
<221>
<223>Probe
<400>9
cacattggcg tgctagcgtt c
<210>10
<211>21
<212>DNA
<213>Artificial Sequence
<220>
<221>
<223>Probe
<400>10
gccctctggg gtgccatccc a
<210>11
<211>21
<212>DNA
<213>Artificial Sequence
<220>
<221>
<223>Probe
<400>11
cacattgcac ttcttgcttt c
<210>12
<211>21
<212>DNA
<213>Artificial Sequence
<220>

<221>
<223>Probe
<400>12
tgtttcagtg acaccatccc a
<210>13
<211>25
<212>DNA
<213>Artificial Sequence
<220>
<221>
<223>Primer
<400>13
gactccatgg caccagcatc aaatc
<210>14
<211>25
<212>DNA
<213>Artificial Sequence
<220>
<221>
<223>Rose3GT16-Spe-I-1 primer
<400>14
acacgtacta gttgtgttcc tgatg
<210>15
<211>29
<212>DNA
<213>Artificial Sequence
<220>
<221>
<223>Rose3GT16-SpeI-3 Primer
<400>15
agtcactagt atgggtggtc acgacgatg
<210>16
<211>24
<212>DNA
<213>Artificial Sequence

<220>
<221>
<223>Rose3GT16-KpnI primer
<400>16
cgttgaggta cctcttgaat ttgg
<210>17
<211>22
<212>DNA
<213>Artificial Sequence
<220>
<221>
<223>pSPB1123-SphI Primer
<400>17
agagttgcat gcaaccgcag tt
<210>18
<211>16
<212>DNA
<213>Artificial Sequence
<220>
<221>
<223>Reverse primer
<400>18
aacagctatg accatg